(19) Europäisches Patentamt
European Patent Office
Office européen des brevets

(11) Numéro de publication: **0 169 141**
**B1**

# (12) FASCICULE DE BREVET EUROPÉEN

(45) Date de publication du fascicule du brevet: **21.09.88**

(51) Int. Cl.⁴: **C 07 C 29/14**, C 07 C 29/136, C 07 C 31/38, C 07 C 31/40

(21) Numéro de dépôt: **85401424.8**

(22) Date de dépôt: **12.07.85**

(54) **Procédé de synthèse du trifluoro-2,2,2 éthanol et de l'alcool hexafluoro-1,1,1,3,3,3 isopropylique.**

(30) Priorité: **18.07.84 FR 8411383**

(43) Date de publication de la demande:
**22.01.86 Bulletin 86/4**

(45) Mention de la délivrance du brevet:
**21.09.88 Bulletin 88/38**

(84) Etats contractants désignés:
**AT BE CH DE FR GB IT LI NL SE**

(56) Documents cité:
**WO-A-81/01843**
**FR-A-2 133 126**
**GB-A-2 087 383**

**Le dossier contient des informations techniques présentées postérieurement au dépôt de la demande et ne figurant pas dans le présent fascicule.**

(73) Titulaire: **ATOCHEM, 4 & 8, Cours Michelet La Défense 10, F-92800 Puteaux (FR)**

(72) Inventeur: **Cheminal, Bernard, Rés. Beauséjour St. Irénée 26, rue Soeur Bouvier, F-69005 Lyon (FR)**
Inventeur: **Mathais, Henri Hameau de Saint-Didier, Villa No. 2 Chemin de l'Indiennerie, F-69370 Saint- Didier- au- Mont- d'Or (FR)**
Inventeur: **Thomarat, Marc, 22 Boulevard de l'Europe, F-69310 Pierre- Bénite (FR)**

(74) Mandataire: **Leboulenger, Jean, ATOCHEM Département Propriété Industrielle, F-92091 Paris la Défense 10 Cédex 42 (FR)**

EP 0 169 141 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

**Description**

La présente invention concerne la fabrication du trifluoro-2,2,2 éthanol et de l'alcool hexafluoro-1,1,1,3,3,3 isopropylique par hydrogénolyse d'hydrates ou d'hémiacétals des composés carbonylés polyfluorés correspondants.

Une importante source industrielle d'alcools primaires fluorés comme le trifluoro-2,2,2 éthanol, qui est utile dans un large domaine d'application telles que la récupération d'énergie (pompes à chaleur à absorption), les produits pharmaceutiques (anesthésiques) et les solvants, provient de la réduction de l'acide correspondant (acide trifluoroacétique en l'occurence) ou d'un dérivé (ester, chlorure d'acide, anhydride, amide), par l'hydrogène en présence d'un catalyseur le plus souvent choisi dans la famille des métaux précieux (rhodium, ruthénium, platine, palladium). Parmi les principales techniques employées, on peut citer l'hydrogénation de l'anhydride trifluoroacétique (brevet US-4 255 594), l'hydrogénation de l'acide trifluoroacétique (brevet US-4 273 947), l'hydrogénation des esters de l'acide trifluoroacétique (brevet EP-36 939), l'hydrogénation du trifluoroacétamide [M. GILMAN, J.A.C.S, 70, 1281-2 (1948)], l'hydrogénolyse du chlorure de trifluoroacétyle (brevet US-3 970 710). Outre l'inconvénient d'une mauvaise tenue du catalyseur dans le temps, ces procédés présentent le désavantage économique de recourir à une oxydation des matières premières (la plupart du temps chlorées) pour accéder à l'acide ou à l'un de ses dérivés, suivie d'une réduction de cet acide en alcool; cette étape supplémentaire grève très sérieusement la rentabilité de ces procédés.

Une autre famille de procédés consiste à hydrogéner le trifluoro-acétaldéhyde (appelé fluoral dans la suite) ou l'un de ses dérivés. Le rendement obtenu par hydrogénation en phase liquide (80°C sous 95 bars) de l'hydrate de fluoral sur nickel de Raney (brevet FR-1 399 290) est médiocre; la durée de vie du catalyseur et la pureté exigée pour la matière première ne sont pas indiquées on peut craindre une réaction parasite, dite réaction haloforme:

$CF_3CHO + NaOH \rightarrow CHF_3 + HCOONa$ (I

avec la soude toujours absorbée sur le catalyseur. On peut citer également le brevet US-2 982 789 qui décrit l'hydrogénation en phase gazeuse du chlorhydrate de fluoral: $CF_3CH(Cl)OH$ provenant d'une première étape d'hydrogénolyse (réaction de ROSENMUND) du chlorure de trifluoroacétyle sur catalyseur au palladium; le catalyseur d'hydrogénation du fluoral, qui consiste de chromite de cuivre déposé sur fluorure de calcium et dont la tenue dans le temps n'est pas mentionnée, fonctionne vers 250°C et ne permet de transformer le fluoral intermédiaire que d'une manière incomplète (environ 60 - 65 %); en outre, le recyclage du chlorhydrate de fluoral non transformé est une opération très risquée à cause de son instabilité thermique, la décomposition:

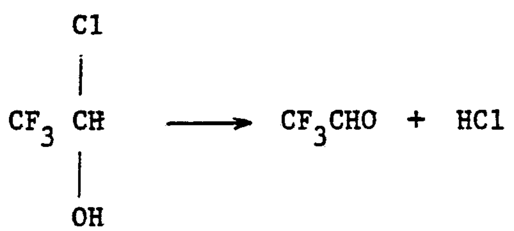
(2)

étant favorisée dès 30°C par une élévation de la température ou une diminution de la pression. On peut citer enfin l'hydrogénation du fluoral en phase gazeuse (brevet US-3 468 964) en présence d'un catalyseur au palladium déposé sur alumine à basse température (température de pointe: 140°C); le rendement modeste en trifluoroéthanol (86 %) et l'obligation de régénérer très souvent le catalyseur à 200°C dans l'oxygène pur, ainsi que l'extrême difficulté de véhiculer le fluoral à l'état pur (polymérisations) rendent le procédé non attractif.

Le brevet FR-2 027 172, relatif à un procédé d'hydrogénation de perhalogénocétones en phase gazeuse sur un catalyseur à base de palladium déposé sur charbon actif, décrit en particulier l'hydrogénation catalytique de l'hexafluoroacétone ($CF_3COCF_3$) en alcool hexafluoro-1,1,1,3,3,3 isopropylique; le rendement obtenu (75 %) et la difficulté de véhiculer la matière première (PEB: -27,4°C/760 torr) rendent le procédé peu économique. Selon le brevet FR-2 479 803 l'hydrogénation catalytique en phase gazeuse de l'hexafluoroacétone en alcool hexafluoro-1,1,1,3,3,3 isopropylique est effectuée sur un catalyseur à base de nickel; la durée de vie du catalyseur n'est pas précisée pour une durée supérieure à 20 heures de fonctionnement continu et ce procédé nécessite surtout l'emploi d'une matière première pure, ce qui est difficile à obtenir avec l'hexafluoroacétone. Dans le brevet FR-1 361 260 l'hydrogénation catalytique de l'hexafluoroacétone en phase gazeuse est réalisée sur un catalyseur à base de chromite de cuivre; les rendements en alcool hexafluoro-1,1,1,3,3,3, isopropylique sont médiocres (environ 40 %) pour une conversion partielle (83 %) de la cétone engagée, ce qui entraîne un recyclage délicat et coûteux.

Le brevet FR-2 133 126 décrit un procédé d'hydrogénation en phase liquide de perfluoroacétone en alcool hexafluoro-isopropylique en présence d'un catalyseur au palladium activé avec une base minérale de type alcalin; le catalyseur ainsi activé permet d'obtenir une conversion partielle (80 à 86 %) de la cétone de départ, mais les temps d'hydrogénation sont longs (environ 7 heures) et l'hexafluoroacétone engagée doit être purifiée ce qui nécessite une distillation préalable sous pression élevée et alourdit le procédé; la durée de vie du

catalyseur n'est pas précisée.

Le brevet FR-2 493 831 décrit un procédé d'hydrogénation de l'hydrate d'hexafluoroacétone en phase gazeuse en présence d'un catalyseur à base de nickel ou de palladium, à l'exclusion du ruthénium ou du platine; ce procédé présente l'inconvénient majeur de nécessiter impérativement l'utilisation d'un hydrate de cétone particulièrement pur et raffiné au moyen d'une technique complexe qui fait l'objet du brevet GB-2 086 891; cette obligation absolue de purifier la matière première jusqu'à un degré de pureté poussé complique notablement le procédé d'obtention d'alcool hexafluoro-1,1,1,3,3,3 isopropylique.

On peut signaler enfin la publication JP-8 388 330 qui décrit un procédé de préparation de l'alcool hexafluoro-1,1,1,3,3,3 isopropylique à partir d'hexafluoroacétone en phase gazeuse (ou éventuellement liquide) en présence d'un catalyseur à base de rhodium déposé sur charbon actif. Son principal inconvénient résulte dans l'utilisation d'un catalyseur très onéreux. De plus, l'indication d'une faible baisse de rendement entre la troisième et la quatrième heure de fonctionnement (exemple 2) de 100 % à 98 % peut prêter à une interprétation variable sur la tenue réelle du catalyseur dans le temps; en effet si cette baisse se poursuit à la même vitesse, ceci signifie que le temps de séjour doit être doublé toutes les 50 heures de marche ce qui n'est pas économiquement viable.

La présente invention permet de remédier à tous ces inconvénients en proposant une technique simple, souple et particulièrement économique pour accéder au trifluoro-2,2,2 éthanol ou à l'alcool hexafluoro-1,1,1,3,3,3 isopropylique, et ceci même à partir d'une matière première brute facile à préparer et à transporter sans nuire ni à la durée de vie du catalyseur, ni au rendement, ni à la productivité.

Le procédé selon l'invention consiste à soumettre un composé de formule générale:

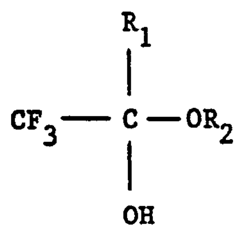

(I)

dans laquelle $R_1$ représente un atome d'hydrogène ou un radical trifluorométhyle et $R_2$ représente un atome d'hydrogène ou un radical alkyle, linéaire ou ramifié, comprenant de 1 à 8 atomes de carbone et éventuellement partiellement fluoré, à une hydrogénolyse en phase liquide en présence d'un catalyseur au palladium déposé sur charbon actif et d'un co-catalyseur constitué par une amine tertiaire aliphatique, ce co-catalyseur n'étant pas indispensable lorsque le composé de formule (I) est l'hémiacétal $CF_3CH(OH)OCH_3$ pur.

Les composés de formule (I), utilisés comme matières premières dans le procédé selon l'invention, peuvent être obtenus, à l'état brut, de manière connue par action de l'eau ($R_2$ = H) ou d'un alcool ($R_2$ = alkyl éventuellement substitué) sur le fluoral ($R_1$ = H) ou l'hexafluoroacétone ($R_1$ = $CF_3$) selon le schéma réactionnel suivant:

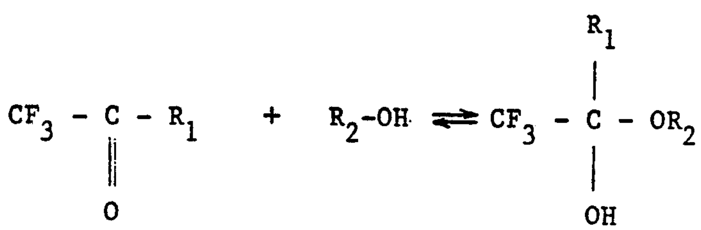

(3)

Comme exemples de radicaux alkyle $R_2$, on peut citer plus particulièrement les radicaux méthyle, éthyle, n. propyle, isopropyle, n. butyle, sec-butyle, n. hexyle, éthyl-2 hexyle, trifluoro-2,2,2 éthyle et hexafluoro-1,1,1,3,3,3 propyl-2.

La stabilité thermique des composés obtenus dépend essentiellement de la température, de la pression et de leur nature chimique. D'une façon générale, les hydrates ($R_2$ = H) sont stables mais difficiles à purifier par distillation. Les hémiacétals ($R_2$ = alkyl) sont d'autant plus stables que le nombre d'atomes de carbone de $R_2$ est faible; leur purification exige parfois une distillation sous léger vide (200 torr) et un temps de séjour court (évaporation en film tombant). A titre d'exemples l'hémiacétal $CF_3$-CH(OH)-$OCH_3$ brut est stable et peut être distillé (point d'ébullition: 96°C/760 torr) à la pression atmosphérique sans décomposition, donc séparé des composés chlorés (plus lourds) et de l'acide chlorhydrique; on obtient alors un produit titrant 99,9 % lorsque l'on évite un temps de séjour trop long (environ quelques minutes) dans le bouilleur de l'appareil à distiller. Par contre, l'hémiacétal $CF_3$-CH(OH)-$OCH_2CF_3$, très instable, ne peut pas être purifié par distillation; on peut néanmoins, conformément à la présente invention, l'utiliser tel quel à l'état brut, c'est-à-dire sous forme de mélange comprenant de 1 à 10 moles pour cent d'hémiacétal chloré $CF_2Cl$-CH(OH)-$OCH_2CF_3$, de 0,1 à 10 moles pour cent d'hémiacétal dichloré $CFCl_2$-CH(OH)-$OCH_2CF_3$ et de 1 à 10 moles pour cent d'acide chlorhydrique.

La teneur en palladium du catalyseur utilisé dans le procédé selon l'invention peut varier entre 0,1 et 10 % en

poids, mais on emploie de préférence un catalyseur à environ 5 % de palladium. La quantité de catalyseur à mettre en oeuvre dépend évidemment de sa teneur en palladium; pour un catalyseur commercial à 5 % de palladium, cette quantité peut varier entre 0,2 et 2,5 % et de préférence entre 0,3 et 1 % par rapport au poids de substrat (hydrate ou hémiacétal) brut mis en oeuvre.

Le catalyseur peut être employé tel quel avec ou sans activation préalable dans le réacteur, avant l'opération d'hydrogénolyse; cette éventuelle activation peut être menée, par exemple, à une température comprise entre 25 et 100°C sous une pression d'hydrogène de 35 à 45 bars.

Après chaque opération d'hydrogénolyse, le catalyseur peut être utilement séparé du mélange réactionnel par décantation puis filtration suivie d'un ou plusieurs lavages à l'eau ou à l'alcool pur synthétisé dans le procédé; on peut également le laisser séjourner (de préférence moins de 48 heures) en contact avec le mélange réactionnel, le laisser décanter, puis séparer le mélange réactionnel par soutirage sous atmosphère d'hydrogène et recommencer une opération suivante sur la même charge catalytique.

Les réactifs (substrat + co-catalyseur éventuel) peuvent être introduits avec le catalyseur au début de la réaction. Cependant, pour éviter une désactivation trop rapide du catalyseur dans le temps, il est préférable d'introduire progressivement le mélange des réactifs dans un mélange constitué d'alcool issu de la synthèse précédente (ou d'alcool purifié) et de catalyseur. On peut suivre la désactivation du catalyseur par l'étude en diffraction X de la dimension des cristallites de palladium formés au cours de la réaction; la vitesse de l'hydrogénolyse est en effet inversement proportionnelle au carré du diamètre des particules actives du catalyseur. Puisque la croissance de ces particules dûe à un phénomène de coalescence correspond à une baisse d'activité de l'espèce catalytique, il est préférable, pour éviter d'accélérer le phénomène de désactivation, d'utiliser un catalyseur amorphe et très divisé dont les particules élémentaires (ou cristallites) ont un diamètre inférieur ou égal à 4 nm, compris de préférence entre 2 et 4 nm.

L'hydrogénolyse peut être effectuée à une température comprise entre 80 et 130°C, de préférence entre 90 et 110°C, et sous une pression comprise entre 20 et 50 bars, de préférence entre 35 et 45 bars.

Dans le cas où, conformément à une modalité préférée du procédé selon l'invention, on introduit progressivement les réactifs (substrat + co-catalyseur éventuel) dans le réacteur d'hydrogénolyse, la durée d'introduction peut varier entre 0,2 et 5 heures, de préférence entre 0,75 et 1,25 heure. Afin d'éviter une désactivation trop rapide du catalyseur, il est particulièrement avantageux que la vitesse d'introduction des réactifs corresponde le plus exactement possible à la vitesse d'hydrogénolyse. Le système de régulation le plus approprié pour parvenir à ce résultat consiste à asservir le débit de la pompe d'injection à la consommation d'hydrogène (elle-même liée au débit ou à la pression d'hydrogène); au fur et à mesure de la lente désactivation du catalyseur dans le temps, on peut ainsi compenser sa légère perte d'activité par une augmentation correspondante de la durée d'introduction des réactifs; ceci augmente considérablement la souplesse du procédé en même temps que son économie.

Le co-catalyseur, constitué par une amine tertiaire aliphatique permet non seulement de faciliter l'ouverture de l'hémiacétal, mais encore de piéger les ions étrangers (plus particulièrement les chlorures et les fluorures) qui risquent de provoquer une accélération de la cristallisation non contrôlée du catalyseur et par suite sa désactivation. Dans la plupart des cas, son élimination du milieu réactionnel, après l'hydrogénolyse, par distillation et son recyclage dans une opération ultérieure sont faciles à mener ; il suffit pour cela de choisir un produit à point d'ébullition adéquat; en outre son caractère basique se prête aisément à une neutralisation (si nécessaire au départ de la distillation) sous forme de sels d'ammonium stables et séparables par distillation, suivie d'une récupération à l'aide d'une base forte (soude ou autre) en fin de distillation.

Les amines tertiaires aliphatiques à utiliser comme co-catalyseurs répondent à la formule:

$$R_3 - \underset{\displaystyle R_4}{\underset{|}{N}} - R_5 \qquad \text{(II)}$$

dans laquelle les symboles $R_3$, $R_4$ et $R_5$ peuvent être identiques ou différents et représentent chacun un radical alkyle éventuellement substitué par un groupe hydroxy. On préfère utiliser la triéthylamine, mais on peut mentionner aussi, à titre d'exemples non limitatifs, la triméthylamine, la tri-n.propylamine, la tributylamine, la diméthyléthylamine, la diméthyl-éthanolamine, la triéthanolamine.

La quantité de co-catalyseur peut varier dans de larges limites en fonction de la pureté du substrat (hydrate ou hémiacétal) soumis à l'hydrogénolyse. Il est évidemment souhaitable de partir d'un substrat aussi pur que possible, mais cela n'est pas toujours réalisable (catalyseur de fluoruration moins performant conduisant à une teneur plus élevée en produits chlorés, réaction secondaire de l'alcool obligeant à absorber le dérivé carbonylé à basse température, ce qui accroît la solubilité de l'acide chlorhydrique, hydrates chlorés et fluorés inséparables par distillation, etc....). La quantité de co-catalyseur est de préférence choisie entre 0 et le nombre de moles de sous-produits chlorés contenus dans la matière première brute, calculé en équivalents $H^+$ et augmenté d'un excés molaire compris entre 5 et 500 %.

Le solvant utilisé peut être n'importe quel solvant organique habituel (cétones aliphatiques, éthers, glycols, solvants chlorés), mais on utilise de préférence l'alcool polyfluoré (trifluoro-2,2,2 éthanol ou alcool hexafluoro-1,1,1,3,3,3 isopropylique) fourni par le procédé selon l'invention; on peut également employer un alcool aliphatique $R_2OH$ ($R_2$ ayant la même signification que ci-dessus). La quantité de solvant à utiliser peut varier de 0 à 100 % par rapport au poids du substrat mis en oeuvre et ne dépend que de la géométrie du réacteur d'hydrogénolyse (en particulier pour assurer une excellente agitation) et de la productivité visée.

Le procédé selon l'invention peut être mis en oeuvre dans un appareil de type classique, c'est-à-dire un autoclave agité par un moyen mécanique adéquat, capable de fonctionner sous une pression de 50 bars et muni d'un dispositif de soutirage de la suspension catalytique et des annexes indispensables (filtre, pompes, régulation de pression d'hydrogène, etc...). La protection contre la corrosion au moyen d'un revêtement intérieur adéquat n'est pas obligatoire, mais peut être envisagée pour éviter que des ions étrangers ($Fe^{2+}$, $Cr^{3+}$, $Ni^{2+}$ ne viennent inhiber la réaction.

L'hydrogénolyse selon l'invention peut également être menée en phase liquide continue sur un lit fixe de catalyseur.

L'alcool polyfluoré produit peut être isolé et purifié par les méthodes conventionnelles telles que la distillation et le séchage sur tamis moléculaire. Sa pureté peut être déterminée par chromatographie en phase gazeuse.

Les exemples suivants illustrent l'invention sans la limiter. Le catalyseur utilisé est un catalyseur standard, fourni par la Société ENGELHARDT; il contient 5 % de palladium déposé sur charbon actif et se présente sous la forme d'une poudre fine dont tous les grains ont un diamètre supérieur à 0,5 um. Dans les tableaux, le terme $C_2$ désigne les composés $CF_xCl_{3-x}CHO$ (x étant égal à 1,2 ou 3).

**Exemple 1**

Hydrogénolyse de $CF_3$-CH(OH)-$OCH_2CH_3$ brut

Dans un autoclave de 0,1 litre muni d'un système d'agitation magnétique, on charge successivement 0,51 g de catalyseur, puis 51 g d'hémiacétal brut contenant (en moles) 94 % de $CF_3$-CH(OH)-O-$CH_2$-$CH_3$ et 6 % de $CF_2Cl$-CH(OH)-O-$CH_2$-$CH_3$ ce qui correspond respectivement à 0,332 mole et 0,021 mole, l'acide chlorhydrique dissous représentant 4,36 $10^{-3}$ mole et l'acide fluorhydrique 0,18 $10^{-30}$ mole. On introduit ensuite 4,7 g de triéthylamine pure (0,0465 mole) ce qui correspond à un excès molaire de 82 % par rapport à la théorie. On ferme le réacteur, purge l'air emmagasiné avec de l'azote et introduit alors une légère pression d'hydrogène, puis on chauffe le mélange réactionnel jusque vers 120°C sous agitation et ajuste la pression d'hydrogène à 43 - 44 bars.

La baisse de pression correspondant à la consommation d'hydrogène est ensuite compensée par des introductions successives d'hydrogène entre 33 et 43 atmosphères. Une fois l'absorption d'hydrogène terminée, on refroidit rapidement le mélange réactionnel puis, après l'avoir dégazé dans un récipient maintenu vers -196°C par de l'azote liquide, on procède à l'ouverture du réacteur et le produit de la réaction mélangé au catalyseur est transféré dans un récipient dans lequel on laisse décanter la suspension catalytique. Après séparation du catalyseur, le produit de l'hydrogénolyse est analysé sur un échantillon. On note le pH d'une solution aqueuse et l'on détermine par analyse minérale les ions chlorures ($C1^-$) et fluorures ($F^-$) formés durant la réaction.

La même charge catalytique est ensuite recyclée au cours de deux opérations successives dans les mêmes conditions opératoires, le même appareillage et avec le même substrat et le même co-catalyseur. Le tableau 1 suivant (page 6) rassemble les résultats obtenus au cours de cette série d'opérations.

**Exemple 2**

Hydrogénolyse de $CF_3$-CH(OH)-$OCH_2CH_3$ brut

On réalise trois opérations en procédant comme à l'exemple 1 aux différences près suivantes:

- l'hémiacétal brut utilisé contient 96 % (en moles) de $CF_3$-CH-(OH)-$OC_2H_5$, 4 % de $CF_2Cl$-CH(OH)-$OC_2H_5$, 1,83.$10^{-3}$ mole d'HCl et 2,74.$10^{-3}$ mole d'HF;
- on utilise seulement 0,17 g de catalyseur et on renouvelle cette charge à chaque opération;
- on fait varier la quantité de triéthylamine: 0,0247 mole pour l'opération 21, 0,0455 mole pour l'opération 22 et 0,0860 mole pour l'opération 23.

Le tableau 2 ci-après rassemble les résultats obtenus au cours de cette série d'opérations.

La comparaison de ces résultats avec ceux obtenus à l'exemple 1 permet d'apprécier l'influence respective du catalyseur et du co-catalyseur sur le déroulement de la réaction.

**Tableau 1**

| OPERATION N° | Durée de la réaction (heures et mn) | Taux de transformation en $CF_3CH_2OH$ % | pH | $Cl^-$ créé (pour 100 moles de $C_2$) | $F^-$ créé (pour 100 moles de $C_2$) |
|---|---|---|---|---|---|
| 11 | 1H | 100 | 10 | 5,25 | 0,69 |
| 12 | 2 H 25 mn | 100 | 9,9 | 4,75 | 0,58 |
| 13 | 6 H 20 mn (réaction incomplète) | 78 | 9,1 | 5,09 | 1,14 |

**Tableau 2**

| OPERATION N° | Mole de co-catalyseur (triéthylamine) | Durée de la réaction (heures & mn) | Taux de transformation en $CF_3CH_2OH$ % | pH | $Cl^-$ créé (pour 100 moles de $C_2$) | $F^-$ créé (pour 100 moles de $C_2$) |
|---|---|---|---|---|---|---|
| 21 | 0,0247 | 4 H 30 mn (réaction incomplète) | 76 | 11,47 | 6,28 | 0,32 |
| 22 | 0,0455 | 2 H 00 mn | 100 | 10 | 6,25 | 0,70 |
| 23 | 0,0860 | 4 H 30 mn | 100 | 10,62 | 6,53 | 0,93 |

**Exemple 3**

Hydrogénolyse de $CF_3$-$CH(OH)_2$ brut

On opère comme à l'exemple 1 avec 0,51 g de catalyseur, 5,35 g de triéthylamine pure (0,053 mole) et 43 g d'hydrate de fluoral brut ayant la composition suivante:

$CF_3$ - $CH(OH)_2$ .......... 0,337 mole
$CF_2Cl$ - $CH(OH)_2$ ......... 0,022 mole
HCl dissous ............. 0,0095 mole
HF dissous ............. $6,88.10^{-5}$ mole

Après 4 heures à 120°C sous environ 35 à 45 bars, la réaction est complète et le rendement en trifluoro-2,2,2 éthanol est quantitatif. On trouve:

$Cl^-$ créé/(mole $C_2$ x 100) = 5,47

$F^-$ créé/(mole $C_2$ x 100) = 1,07

**Exemple 4**

Hydrogénolyse de $CF_3$-$CH(OH)$-$O$-$CH_2$-$CF_3$ brut

On opère comme à l'exemple 1 avec 0,43 g de catalyseur, 5,35 g de triéthylamine pure (0,053 mole) et 59,4 g de l'hémiacétal brut ayant la composition suivante:

| | |
|---|---|
| $CF_3$-CH(OH)-O-$CH_2CF_3$ | ..... 0,300 mole |
| $CF_2Cl$-CH(OH)-O-$CH_2CF_3$ | ..... 0,018 mole |
| HCl dissous ......... | ...... 0,012 mole |
| HF dissous ........... | ...... 0,0003 mole |

Après une heure à 120°C sous 35 à 50 bars, le taux de transformation en trifluoro-2,2,2 éthanol est de 100 %. Le pH d'une solution aqueuse du produit d'hydrogénolyse est égal à 9,7 et on trouve:

$Cl^-$ créé/(mole $C_2$ x 100) = 7,26

$F^-$ créé/(mole $C_2$ x 100) = 0,60

Une seconde opération effectuée avec le catalyseur issu de l'opération précédente fournit les résultats suivants:

| | |
|---|---|
| - Taux de transformation en $CF_3CH_2OH$ après 5 heures de réaction | 89% |
| - pH | 6,0 |
| - $Cl^-$ créé | 7,00 |
| - $F^-$ créé | 0,72 |

**Exemple 5**

Hydrogénolyse de $CF_3$-CH(OH)-$OCH_2CF_3$ brut

Dans le même autoclave qu'à l'exemple 1, on charge 20,9 g de trifluoro-2,2,2 éthanol pur et 0,44 g de catalyseur, puis on chauffe ce mélange à 100°C sous une pression de 40 bars d'hydrogène pendant 30 minutes (activation).

Tout en maintenant cette température et cette pression d'hydrogène, on introduit ensuite progressivement (en 72 minutes) un mélange de 1,82 g de triéthylamine (0,018 mole) et de 61 g d'un hémiacétal brut ayant la composition suivante:

| | |
|---|---|
| $CF_3$-CH(OH)-O-$CH_2$-$CF_3$ | 0,292 mole |
| $CF_2Cl$-CH(OH)-O-$CH_2$-$CF_3$ | 0,008mole |
| $CFCl_2$-CH(OH)-O-$CH_2CF_3$ | 0,0009 mole |
| HCl dissous | 0,0068 mole |
| HF dissous | $1{,}7.10^{-5}$ mole |

Après la fin de l'introduction, on poursuit l'alimentation en hydrogène jusqu'à la fin de la réaction matérialisée par l'absence de consommation d'hydrogène.

Après refroidissement rapide du mélange réactionnel par trempe du réacteur dans de l'azote liquide, on récupère rapidement le catalyseur en ouvrant le réacteur et en séparant le solide sur un filtre de 0,5 micron. On lave 8 fois le catalyseur à l'eau et on le sèche pendant 20 minutes sous 200 torrs à température ambiante, puis 2 heures sous 1 torr à température ambiante et enfin 2 heures sous 1 torr à une température d'environ 95° C.

Le catalyseur ainsi traité est alors recyclé au cours de deux autres opérations successives dans les mêmes conditions opératoires. Le tableau 3 ci-après rassemble les résultats obtenus dans ces trois opérations.

**Tableau 3**

| OPERATION N° | Durée de la réaction (heures et mn) | Taux de transformation en $CF_3CH_2OH$ % | pH | Vitesse moyenne moles $C_2$/(h x at.g Pd) |
|---|---|---|---|---|
| 51 | 1 H 24 mn | 100 | 10 | 1 250 |
| 52 | 1 H 20 mn | 100 | 9,9 | 1 240 |
| 53 | 1 H 30 mn | 100 | 9,9 | 1 150 |

Si l'on tient compte de la perte mécanique de catalyseur (environ 5 %) on peut estimer à 4 % la perte d'activité du catalyseur au cours de ces trois opérations successives, ce qui est faible.

**Exemple 6**

Hydrogénolyse de $CF_3$-CH(OH)-O-$CH_2CF_3$ brut

Dans le même autoclave qu'à l'exemple 1, on réalise sept opérations successives de la façon suivante:

Opération n° 61

On charge 20,9 g de trifluoro-2,2,2 éthanol pur et 0,43 g de catalyseur, puis on chauffe ce mélange à 100°C sous une pression de 40 bars d'hydrogène pendant 30 minutes. On introduit ensuite progressivement (en 72 minutes, un mélange de 1,82 g de triéthylamine et de 61 g du même hémiacétal brut qu'à l'exemple 5, tout en maintenant la température à 100°C environ et la pression d'hydrogène à 40 bars. Après la fin de l'introduction, on poursuit l'alimentation en hydrogène (35 à 45 bars) jusqu'à la fin de la réaction, puis on refroidit rapidement le mélange réactionnel en trempant le réacteur dans l'azote liquide. On laisse ensuite décanter pendant 20 heures, puis on soutire le liquide réactionnel à l'aide d'un tube plongeant débouchant à 8 mm au-dessus du fond du réacteur.

Opération n° 62

On répète l'opération n° 61, mais en utilisant le catalyseur maintenu dans le réacteur à la fin de ladite opération.

Opération n° 63

On répète l'opération n° 61 en utilisant le catalyseur maintenu dans le réacteur à la fin de l'opération n° 62. En outre, après le refroidissement, on laisse décanter pendant 75 heures avant de procéder au soutirage.

Opération n° 64

On répète l'opération n° 61 avec le catalyseur provenant de l'opération n° 63. Cependant, on procède à un refroidissement lent (environ 4 heures) du mélange réactionnel qu'on laisse ensuite décanter pendant 20 heures.

Opération n° 65

On répète l'opération n° 64 avec le catalyseur provenant de ladite opération.

Opération n° 66

On répète l'opération n° 64 avec le catalyseur provenant de l'opération n° 65, mais sans l'activer préalablement (pas de chauffage à 100°C sous pression d'hydrogène) avant l'introduction continue des réactifs.

Opération n° 67

On répète l'opération n° 64 avec le catalyseur issu de l'opération n° 66, mais avec activation préalable.

Le tableau 4 suivant rassemble les résultats obtenus au cours de ces sept opérations.

**Tableau 4**

| OPERATION N° | Durée de la réaction (heures et mn) | Taux de transformation en $CF_3CH_2OH$ % | pH | Vitesse moyenne moles $C_2$ / (h x at.g Pd) |
|---|---|---|---|---|
| 61 | 1 H 30 mn | 100 | 10 | 1400 |
| 62 | 1 H 24 mn | 100 | 10 | 1390 |
| 63 | 1 H 24 mn | 100 | 10,1 | 1390 |
| 64 | 1 H 55 mn | 100 | 10,1 | 1210 |
| 65 | 2 H 15 mn | 100 | 10,1 | 1147 |
| 66 | 3 H 10 mn | 100 | 9,9 | 795 |
| 67 | 3 H 00 mn | 100 | 9,5 | 1028 |

**Exemple 7**

Hydrogénolyse de $CF_3$-CH(OH)-$OCH_3$ pur

Dans le même autoclave qu'à l'exemple 1, on charge 20 g de trifluoro-2,2,2 éthanol pur et 0,60 g de catalyseur, puis on chauffe ce mélange à 100°C sous une pression de 40 bars d'hydrogène pendant 30 minutes (activation). Tout en maintenant cette température et cette pression d'hydrogène, on introduit ensuite en une heure un mélange de 0,63 g de triéthylamine et 54,6 g d'hémiacétal $CF_3$-CH(OH) -$OCH_3$ pur (pureté supérieure à 99,5 %).

On récupère le catalyseur en opérant comme à l'exemple 5 et le réemploie dans trois autres opérations effectuées de la même façon.

Le tableau 5 rassemble les résultats ainsi obtenus.

**Tableau 5**

| OPERATION N° | Durée de la réaction (heures et mn) | Taux de transformation en en $CF_3CH_2OH$ % | pH | Vitesse moyenne moles $C_2$/(h x at.g Pd) |
|---|---|---|---|---|
| 71 | 1 H 20 mn | 100 | 9,9 | 1 372 |
| 72 | 1 H 35 mn | 100 | 10,0 | 1 237 |
| 73 | 1 H 32 mn | 100 | 10,0 | 1 200 |
| 74 | 1 H 55 mn | 100 | 9,8 | 1 032 |

**Exemple 8**

Hydrogénolyse de $CF_3$-CH(OH)-$OCH_3$ pur

On opère comme à l'exemple 1, mais avec 0,43 g du catalyseur, 0,455 g de triéthylamine ($4,5.10^{-3}$ mole) et 39 g d'un hémiacétal ayant la composition suivante:

| | |
|---|---|
| $CF_3$-CH(OH)-$OCH_3$ | 99,2 %, soit 0,298 mole |
| $CF_2Cl$-CH(OH)-$OCH_3$ | 0,1 %, soit 0,002 mole |
| autres impuretés | 0,7 % |

Après 30 minutes à 100°C sous 35 à 45 bars, le taux de transformation en trifluoro-2,2,2 éthanol est de 100 l. Le pH d'une solution aqueuse du produit d'hydrogénolyse est de 9,7 et on trouve:

$Cl^-$ créé/(mole $C_2$ x 100) = 0,26

$F^-$ créé/(mole $C_2$ x 100) = 0,10

Après séparation par décantation à l'extérieur du réacteur, la charge de catalyseur est utilisée pour une seconde opération réalisée dans les mêmes conditions. Après 5 heures de réaction, on obtient les résultats suivants:

| | |
|---|---|
| Taux de transformation | env. 100 % |
| pH | 8,9 |
| $Cl^-$ créé | 0,23 |
| $F^-$ créé | 0,16 |

**Exemple 9**

On opère à l'exemple 5, mais en remplaçant la triéthylamine par la même proportion molaire de diméthyléthylamine. Les résultats obtenus sont résumés dans le tableau suivant:

**Tableau 6**

| OPERATION N° | Durée de la réaction (heures et mn) | Taux de transformation en $CF_3CH_2OH$ % | pH | Vitesse moyenne moles $C_2$/(h x at.g Pd) |
|---|---|---|---|---|
| 91 | 1 H 7 mn | 100 | 9,6 | 1 310 |
| 92 | 1 H 12 mn | 100 | 9,7 | 1 263 |
| 93 | 1 H 7 mn | 100 | 9,8 | 1 400 |

**Exemple 10**

Hydrogénolyse de 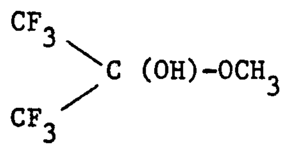 brut

Dans le même autoclave qu'à l'exemple 1, on charge 21 g d'alcool hexafluoroisopropylique et 0,42 g de catalyseur, puis on chauffe ce mélange à 100° C sous une pression de 40 bars d'hydrogène pendant 30 minutes.

On introduit ensuite en environ 70 minutes un mélange de 10,7 g (0,106 mole) de triéthylamine et de 56 g (0,275 mole) d'un hémiacétal de formule brute $C_4H_4F_{5,67}Cl_{0,33}O_2$ contenant $3,25.10^{-3}$ mole d'acide chlorhydrique et $2,25.10^{-4}$ mole d'acide fluorhydrique.

Après 6 heures de réaction à 100°C sous 31 à 46 bars, le taux de transformation en alcool hexafluoroisopropylique est voisin de 100 %. On trouve:

$Cl^-$ créé/(mole $C_3$ x 100) = 25,5

$F^-$ crée/(mole $C_3$ x 100) = 18,8

**Revendications**

1. Procédé de synthèse du trifluoro-2,2,2 éthanol et de l'alcool hexafluoro-1,1,1,3,3,3 isopropylique par hydrogénolyse catalytique d'un composé de formule:

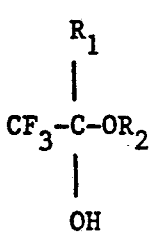 (I)

dans laquelle

$R_1$ est un atome d'hydrogène ou un radical trifluorométhyle et

$R_2$ représente un atome d'hydrogène ou un radical alkyle, linéaire ou ramifié, comprenant de 1 à 8 atomes de carbone et éventuellement partiellement fluoré, caractérisé en ce que l'on opère en phase liquide en présence d'un catalyseur au palladium déposé sur charbon actif et d'une amine tertiaire aliphatique comme co-catalyseur, ce dernier n'étant pas indispensable lorsque le composé de formule (I) est l'hémiacétal $CF_3CH(OH)OCH_3$ pur.

2. Procédé selon la revendication 1, dans lequel on introduit progressivement un mélange d'un composé de formule (I) et d'un co-catalyseur dans une suspension du catalyseur dans le polyfluoroalcool $CF_3$-CH(OH)-$R_1$.

3. Procédé selon la revendication 1 ou 2, dans lequel, après cessation de la réaction, on refroidit rapidement le mélange réactionnel, sépare le catalyseur et le réutilise dans une opération ultérieure.

4. Procédé selon la revendication 3, dans lequel on sépare le catalyseur par filtration, le lave à l'eau et le

sèche avant réutilisation.

5. Procédé selon la revendication 3, dans lequel, après refroidissement du mélange réactionnel, on laisse décanter le catalyseur, puis soutire le liquide réactionnel et réutilise tel quel le catalyseur maintenu dans le réacteur.

6. Procédé selon l'une des revendications 1 à 5, dans lequel on emploie un composé de formule (I) à l'état brut.

7. Procédé selon l'une des revendications 1 à 6, dans lequel on opère à une température allant de 80 à 130° C, de préférence entre 90 et 110° C.

8. Procédé selon l'une des revendications 1 à 7, dans lequel on opère sous une pression de 20 à 50 bars, de préférence entre 35 et 45 bars.

9. Procédé selon l'une des revendications 1 à 8, dans lequel la teneur en palladium du catalyseur varie de 0,1 à 10 % en poids et est, de préférence, égale à environ 5 %.

10. Procédé selon l'une des revendications 1 à 9, dans lequel le catalyseur est préalablement activé à une température comprise entre 25 et 100° C sous une pression d'hydrogène de 35 à 45 bars.

11. Procédé selon l'une des revendications 1 à 10, dans lequel la quantité de catalyseur, exprimée pour un catalyseur à 5 % de palladium, est comprise entre 0,2 et 2,5 %, de préference entre 0,3 et 1 %, par rapport au poids d'hydrate ou d'hémiacétal mis en oeuvre.

12. Procédé selon l'une des revendications 1 à 9, dans lequel la quantité de co-catalyseur est comprise entre 0 et le nombre de moles de sous-produits chlorés contenus dans le composé de formule (I) brut, calculé en équivalents H+ et augmenté d'un excès molaire compris entre 5 et 500 %.

**Patentansprüche**

1. Verfahren zur Synthese von 2,2,2-Trifluorethanol und von 1,1,1,3,3,3-Hexafluorisopropylalkohol durch katalytische Hydrogenolyse einer Verbindung der Formel:

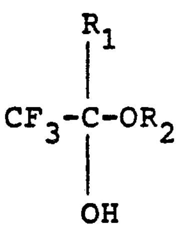

(I)

in der

$R_1$ ein Wasserstoffatom oder ein Trifluormethylrest ist und

$R_2$ ein Wasserstoffatom oder einen Alkylrest darstellt, der linear oder verzweigt ist und 1 bis 8 Kohlenstoffatome enthält und gegebenenfalls teilweise fluoriert ist, dadurch gekennzeichnet, daß man in flüssiger Phase in Gegenwart eines Katalysators von Palladium auf Aktivkohle sowie eines tertiären aliphatischen Amins als Cokatalysator arbeitet, wobei letzteres nicht unbedingt notwendig ist, wenn die Verbindung der Formel (5) das reine Hemiacetal $CF_3$-CH(OH)$OCH_3$ ist.

2. Verfahren nach Anspruch 1, bei dem man nach und nach ein Gemisch einer Verbindung der Formel (I) und eines Cokatalysators zu einer Suspension des Katalysators in dem Polyfluoroalkohol $CF_3$-CH(OH)-$R_1$ zugibt.

3. Verfahren nach Anspruch 1 oder 2, bei dem man nach Beendigung der Reaktion das Reaktionsgemisch schnell abkühlt, den Katalysator abtrennt und diesen in einem späteren Arbeitsgang wieder verwendet.

4. Verfahren nach Anspruch 3, bei dem man den Katalysator durch Filtration abtrennt, diesen mit Wasser wäscht und dann vor der Wiederverwendung trocknet.

5. Verfahren nach Anspruch 3, bei dem man nach dem Abkühlen des Reaktionsgemischs den Katalysator sich absetzen läßt, die Reaktionsflüssigkeit abzieht und den im Reaktor zurückbleibenden Katalysator so wiederverwendet.

6. Verfahren nach einem der Ansprüche 1 bis 5, bei dem man eine Verbindung der Formel (5) im Rohzustand einsetzt.

7. Verfahren nach einem der Ansprüche 1 bis 6, bei dem man bei einer Temperatur von 80 bis 130° C arbeitet, vorzugsweise zwischen 90 und 110° C.

8. Verfahren nach einem der Ansprüche 1 bis 7, bei dem man bei einem Druck von 20 bis 50 bar, vorzugsweise zwischen 35 und 45 bar arbeitet.

9. Verfahren nach einem der Ansprüche 1 bis 8, bei dem der Gehalt des Katalysators an Palladium zwischen 0,1 und 10 Gew.-% variiert und vorzugsweise ungefähr 5 % beträgt.

10. Verfahren nach einem der Ansprüche 1 bis 9, bei dem der Katalysator vorher bei einer Temperatur zwischen 25 und 100° C bei einem Wasserstoffdruck von 35 bis 45 bar aktiviert wird.

11. Verfahren nach einem der Ansprüche 1 bis 10, bei dem die Menge an Katalysator angegeben für einen Katalysator mit 5 % Palladium zwischen 0,2 und 2,5 %, vorzugsweise zwischen 0,3 und 1 % beträgt bezogen auf

das Gewicht des umgesetzten Hydrats oder Hemiacetals.

12. Verfahren nach einem der Ansprüche 1 bis 9, bei dem die Menge des Cokatalysators zwischen 0 und der Anzahl der Mole von in der rohen Verbindung der Formel (I) enthaltenen chlorierten Nebenprodukten liegt, berechnet als H+-Äquivalente und erhöht um einen molaren Überschuß von zwischen 5 und 500 %.

## Claims

1. Process for the synthesis of 2,2,2-trifluoroethanol and 1,1,1,3,3,3-hexafluoroisopropyl alcohol by catalytic hydrogenolysis of a compound of formula:

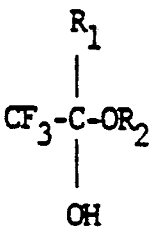

(I)

in which

$R_1$ is a hydrogen atom or a trifluoromethyl radical and

$R_2$ denotes a hydrogen atom or a straight-chain or branched alkyl radical containing from 1 to 8 carbon atoms and optionally partly fluorinated, characterized in that it is carried out in liquid phase in the presence of a catalyst containing palladium deposited on activated charcoal and of a tertiary aliphatic amine as a cocatalyst, the latter not being indispensable when the compound of formula (I) is the pure hemiacetal $CF_3CH(OH)OCH_3$.

2. Process according to Claim 1, in which a mixture of a compound of formula (I) and of a cocatalyst is added gradually to a suspension of the catalyst in the polyfluoroalcohol $CF_3$-CH(OH)-$R_1$.

3. Process according to Claim 1 or 2, in which, after the reaction has ceased, the reaction mixture is rapidly cooled and the catalyst is separated off and reused in a subsequent operation.

4. Process according to Claim 3, in which the catalyst is separated off by filtration and is washed with water and dried before reuse.

5. Process according to Claim 3, in wich, after cooling of the reaction mixture, the catalyst is allowed to separate by gravity, and then the reaction liquid is withdrawn and the catalyst kept in the reactor is reused as such.

6. Process according to one of Claims 1 to 5, in which a compound of formula (I) is used in a crude state.

7. Process according to one of Claims 1 to 6, in which the operation is carried out at a temperature ranging from 80 to 130°C, preferably between 90 and 110°C.

8. Process according to one of Claims 1 to 7, in which the operation is carried out at a pressure of 20 to 50 bars, preferably between 35 and 45 bars.

9. Process according to one of Claims 1 to 8, in which the palladium content of the catalyst varies from 0.1 to 10 % by weight and is, preferably, equal to approximately 5 %.

10. Process according to one of Claims 1 to 9, in which the catalyst is activated beforehand at a temperature of between 25 and 100°C at a hydrogen pressure of 35 to 45 bars.

11. Process according to one of Claims 1 to 10, in which the quantity of catalyst, expressed for a catalyst containing 5 % of palladium, is between 0.2 and 2.5 %, preferably between 0.3 and 1 %, relative to the weight of hydrate or hemiacetal employed.

12. Process according to one of Claims 1 to 9, in which the quantity of cocatalyst is between 0 and the number of moles of chlorinated by-products present in the crude compound of formula (I), calculated in H+ equivalents and increased by a molar excess of between 5 and 500 %.